# EUROPEAN PATENT APPLICATION

(11) **EP 4 495 103 A1**
(43) Date of publication of application: **22.01.2025**
(21) Application number: 23770897.9
(22) Date of filing: 16.03.2023
(51) Int. Cl.: C07D 233/58, C08B 3/06, C08B 3/22

(54) **METHOD FOR RECOVERING IONIC LIQUID**

(30) Priority: 16.03.2022 WO PCT/JP2022/011993
(71) Applicant: National University Corporation Kanazawa University, Ishikawa 920-1192 (JP); Daicel Corporation, Osaka-shi, Osaka 530-0011 (JP)
(72) Inventor: WADA, Naoki, Kanazawa-shi, Ishikawa 920-1192 (JP); TAKAHASHI, Kenji, Kanazawa-shi, Ishikawa 920-1192 (JP); OKUBO, Yuto, Tokyo 108-8230 (JP); WATANABE, Susumu, Tokyo 108-8230 (JP); WATANABE, Hitoshi, Tokyo 108-8230 (JP)
(74) Representative: Grünecker Patent- und Rechtsanwälte PartG mbB
(86) International application number: PCT/JP2023/010485
(87) International publication number: WO 2023/176944

(57) **Abstract**

Provided is a method for easily and efficiently recovering an ionic liquid from a mixed liquid containing the ionic liquid and an organic acid. In the method for recovering the ionic liquid from the mixed liquid containing the ionic liquid and the organic acid, the ionic liquid is water-soluble, and the method includes blending at least one type selected from the group consisting of water and an organic solvent (excluding the organic acid) in the mixed liquid, and separating the organic acid by extraction.

## Description

### Technical Field

The present disclosure relates to a method for recovering an ionic liquid by extraction.

### Background Art

In recent years, ionic liquids have been proposed as solvents for dissolving biomass containing a polysaccharide, such as cellulose or lignocellulose, (hereinafter, which may be referred to as "polysaccharide-containing biomass"), and techniques for derivatizing the polysaccharide under a homogeneous reaction system by using the ionic liquids have been introduced. The ionic liquids have extremely low volatility, pose no risks such as contamination and ignition resulting from volatilization, and have strong ability of dissolving cellulose or the like. Thus, research and development are underway for using the ionic liquids as solvents in processing of polysaccharides.

For example, Example 1 of Patent Document 1 describes that 120 mg of bagasse (sugar cane residue) was dissolved in 4 g of 1-ethyl-3-methylimidazolium acetate that is an ionic liquid (bagasse concentration in the ionic liquid: 3 wt.%), the solution was vacuum-dried under stirring conditions at 80°C overnight, then 4 mL of isopropenyl acetate was added to the reaction system, and the resulting reaction solution was reprecipitated with methanol and filtered to obtain a polysaccharide derivative (cellulose acetate) in a solid state.

Herein, since the ionic liquid is very expensive, a method for recovering and reusing the ionic liquid after the completion of the reaction has been studied. For example, Patent Document 2 describes a method for separating and recovering an ionic liquid from a mixed solution, in which two or more solutes including the ionic liquid are dissolved, by using an ion exchange membrane. Moreover, Patent Document 3 describes a method for separating and recovering an ionic liquid, which has, as a cation component, a quaternary ammonium skeleton having an alkoxyalkyl group or a nitrogen-containing five-membered heterocyclic skeleton having an alkoxyalkyl group, and has an amino group as an anion component, by crystallizing and then washing with a solvent such as ethyl acetate. Furthermore, Patent Document 4 describes a method for separating and recovering an ionic liquid containing impurities by dissolving the ionic liquid in a mixed solvent of a polar aprotic organic solvent and a nonpolar organic solvent, and then lowering the temperature of the solution to crystallize the ionic liquid from the solution. Further, Patent Document 5 describes a method for purifying an ionic liquid by partially crystallizing the ionic liquid from a melt thereof and separating the crystal product from the remaining melt.

### Citation List

### Patent Document

Patent Document 1: WO 2016/068053
Patent Document 2: JP 2015-96255 A
Patent Document 3: JP 2012-144441 A
Patent Document 4: JP 2010-184902 A
Patent Document 5: JP 2008-523005 A

### Summary of Invention

### Technical Problem

However, when cellulose acetate is produced from the polysaccharide-containing biomass as in Patent Document 1, an esterification reaction of the polysaccharide-containing biomass is performed in the presence of the polysaccharide-containing biomass and an esterifying agent serving as raw materials and an ionic liquid serving as a solvent. However, an organic acid derived from the esterifying agent is produced as a by-product of the reaction. Thus, the reaction solution after the esterification reaction is a mixture containing the organic acid and the ionic liquid. For such a mixture, although the reason for this is not clear, it has been difficult to separate and recover the ionic liquid from the mixed liquid probably because of the interaction between the organic acid and the ionic liquid.

Therefore, an object of the present disclosure is to provide a method for easily and efficiently recovering an ionic liquid from a mixed liquid containing the ionic liquid and an organic acid.

### Solution to Problem

As a result of intensive studies for solving the above problems, the inventors of the present disclosure have found that an ionic liquid can be easily and efficiently recovered by blending at least one type selected from the group consisting of water and an organic solvent in a mixed liquid containing the ionic liquid and an organic acid, followed by extraction. The invention according to the present disclosure was completed based on these findings.

That is, the present disclosure provides a method for recovering an ionic liquid from a mixed liquid containing the ionic liquid and an organic acid, in which the ionic liquid is water-soluble, and the method includes blending at least one type selected from the group consisting of water and an organic solvent (excluding an organic acid) in the mixed liquid, and separating the organic acid by extraction.

The ionic liquid preferably contains an imidazolium cation or a quaternary ammonium cation as a cation component.

The ionic liquid preferably contains a carboxylate anion as an anion component.

The organic acid is preferably carboxylic acid.

The organic solvent is preferably at least one type selected from the group consisting of ethers, ketones, esters, aryls, alkanes, and cycloalkanes.

The organic solvent is preferably at least one type selected from the group consisting of ethers, ketones, and esters.

The organic acid is preferably a C₁₋₇ carboxylic acid.

### Advantageous Effects of Invention

According to the method for recovering an ionic liquid of the present disclosure, it is possible to easily and efficiently recover the ionic liquid from a mixed liquid containing the ionic liquid and an organic acid.

### Description of Embodiments

A method for recovering an ionic liquid according to an embodiment of the present disclosure is a method for recovering an ionic liquid from a mixed liquid containing the ionic liquid and an organic acid, in which the ionic liquid is water-soluble, and the method includes blending at least one type selected from the group consisting of water and an organic solvent (excluding an organic acid) in the mixed liquid, and separating the organic acid by extraction. Hereinafter, the method for recovering an ionic liquid of the present disclosure may be referred to as a "method of the present disclosure." Moreover, at least one type selected from the group consisting of water and an organic solvent (excluding an organic acid) may be described as water and/or an organic solvent (excluding an organic acid).

### Mixed Liquid

Hereinafter, a mixed liquid (A) and a mixed liquid (B) will be described. In the present specification, a mixed liquid containing an ionic liquid and an organic acid is referred to as a mixed liquid (B). Moreover, in the present specification, a mixed liquid containing an ionic liquid, an organic acid, water, and an organic solvent (excluding an organic acid) is referred to as a mixed liquid (A). The mixed liquid (A) contains an ionic liquid, an organic acid, water, and an organic solvent (excluding an organic acid) and is not particularly limited as long as the ionic liquid is water-soluble. However, the mixed liquid (A) is preferably produced by blending water and/or an organic solvent (excluding an organic acid) in the mixed liquid (B) containing the ionic liquid and the organic acid. The mixed liquid (B) contains an ionic liquid and an organic acid and is not particularly limited as long as the ionic liquid is water-soluble. For example, the mixed liquid (B) may be a mixed liquid (hereinafter, which may be referred to as a "mixed liquid (B1)") containing a reaction product obtained when a specific raw material is esterified with an esterifying agent or the like in the ionic liquid used as a solvent. The mixed liquid (B1) contains, for example, an esterified product as the target product (an esterified product of the raw material), an organic acid derived from the esterifying agent, and an ionic liquid. Moreover, the mixed liquid (B1) may further contain the unreacted raw material, the unreacted esterifying agent, a by-product other than the organic acid, a solvent other than the ionic liquid, and the like. Furthermore, the mixed liquid (B1) may be obtained by removing a part or all of components other than the ionic liquid and the organic acid by a known or commonly used method. That is, the mixed liquid (B1) is only required to contain at least the organic acid derived from the esterifying agent and the ionic liquid.

The mixed liquid (B1) in a case where the raw material is a polysaccharide will be described below. That is, the mixed liquid (B) is a mixed liquid (hereinafter, which may be referred to as a "mixed liquid (B2)") obtained when the polysaccharide is esterified with an esterifying agent in the ionic liquid as a solvent. The mixed liquid (B2) contains, for example, an esterified product of the polysaccharide which is the target product, an organic acid derived from the esterifying agent, and the ionic liquid. The mixed liquid (B2) may further contain the unreacted polysaccharide, the unreacted esterifying agent, a by-product other than the organic acid, a solvent other than the ionic liquid, and the like. In addition, the mixed liquid (B2) may be obtained by removing a part or all of components other than the ionic liquid and the organic acid by a known or commonly used method.

Summary of the above is as follows:
the mixed liquid (B1) is an example of a specific embodiment of the mixed liquid (B) and is a mixed liquid (B) containing a reaction product obtained when a specific raw material is esterified with an esterifying agent or the like in the ionic liquid used as a solvent;
the mixed liquid (B2) is an example of a more specific embodiment of the mixed liquid (B1) and is a mixed liquid (B) in a case where the raw material, which forms an esterified product by an esterifying agent, is a polysaccharide; and
the mixed liquid (B) is a conceptual mixed liquid encompassing the mixed liquid (B1) and the mixed liquid (B2). Herein, in the present embodiment, the mixed liquid (B) is not limited to the mixed liquid (B1) or the mixed liquid (B2).

The raw material, the esterifying agent, the ionic liquid, and the solvent other than the ionic liquid in a case where the mixed liquid (B) is the mixed liquid (B1) will be described in detail below.

### Raw Material

First, the raw material will be described. The raw material means a starting material to be esterified by an esterifying agent.

The raw material is preferably a polysaccharide. Herein, as previously mentioned, when the raw material is a polysaccharide, the mixed liquid (B1) indicates the mixed liquid (B2).

The polysaccharide may be biomass containing a polysaccharide (polysaccharide-containing biomass). The polysaccharide-containing biomass is not particularly limited as long as the biomass contains a polysaccharide. Examples thereof include bagasse (sugar cane residue): wood, such as kenaf, Japanese cedar, eucalyptus, Japanese red pine, poplar, lauan, Japanese cypress, monarch birch, and Sitka spruce; shells of crustaceans, such as crabs and shrimps; cereals, such as rice, wheat, corn, and sorghum; tubers and roots, such as a potato, a sweet potato, and cassava; and other cellulosic plant-derived raw materials (pulp waste liquid; rice straws; chaff; fruit fibers; fruit kernel shells, such as those of ginkgo nuts; and empty fruit bunches. In addition, pulp or the like obtained by refining these biomasses can also be used. Note that, the polysaccharide-containing biomass may be subjected to various pretreatments, such as cutting and drying, as necessary and undergo separation and extraction of polysaccharide (e.g., cellulose) to be transformed into a polysaccharide form. One type of the polysaccharide may be used, or two or more types of the polysaccharides may be used.

The polysaccharide is not particularly limited, and examples thereof include cellulose, hemicellulose, xylan, mannan, glucomannan, glucuronoxylan, starch, amylose, amylopectin, glycogen, dextrin, pectin, chitin, chitosan, agarose, carrageenan, isolichenan, laminaran, lichenan, glucan, inulin, levan, fructan, galactan, arabinan, pentosan, alginic acid, pectic acid, protuberic acid, colominic acid, porphyran, fucoidan, ascophyllan, locust bean gum, guar gum, tamarind gum, tara gum, and gum arabic.

### Esterifying Agent

Next, an esterifying agent will be described. The esterifying agent is a compound for esterifying a raw material such as a polysaccharide.

The esterifying agent is not particularly limited, and a compound corresponding to the type of a target esterified product can be appropriately selected and used. In particular, the esterifying agent is preferably one or more selected from the group consisting of a chain ester compound, a cyclic ester compound, an unsaturated aldehyde, a saturated aldehyde, an acid halide, an acid anhydride, and an allyl alcohol. Among them, an acid anhydride is preferred from the viewpoint of handleability and the yield of the resulting esterified product. One type of the esterifying agent can be used, or two or more types of the esterifying agents can be used in combination.

The chain ester compound is not particularly limited, and examples thereof include a compound, such as carboxylic acid alkyl ester (e.g., methyl acetate) and carboxylic acid alkenyl ester (e.g., isopropenyl acetate and vinyl acetate).

The cyclic ester compound is not particularly limited, and examples thereof include lactones, such as β-propiolactone, δ-valerolactone, γ-butyrolactone,ε-caprolactone, α,α-dimethyl-β-propiolactone, β-ethyl-δ-valerolactone, α-methyl-ε-caprolactone, β-methyl-ε-caprolactone, γ-methyl-ε-caprolactone, and 3,3,5-trimethyl-ε-caprolactone; and lactides, such as glycolide and lactide.

The unsaturated aldehyde is not particularly limited, and examples thereof include aromatic aldehydes, such as 2-butenal, 2-hexenal, 2-decenal, 2-undecenal, 2-dienal, 2,4-heptadienal, 2,4-decadienal, cinnamaldehyde, and benzaldehyde.

The saturated aldehyde is not particularly limited, and examples thereof include propanal, hexanal, octanal, and nonanal.

The acid halide is not particularly limited, and examples thereof include carboxylic acid halide, such as carboxylic acid fluoride, carboxylic acid chloride, carboxylic acid bromide, and carboxylic acid iodide. Specific examples of the carboxylic acid halide include acetyl fluoride, acetyl chloride, acetyl bromide, acetyl iodide, propionyl fluoride, propionyl chloride, propionyl bromide, propionyl iodide, butyryl fluoride, butyryl chloride, butyryl bromide, butyryl iodide, benzoyl fluoride, benzoyl chloride, benzoyl bromide, and benzoyl iodide.

The acid anhydride is not particularly limited, and examples thereof include acetic anhydride, propionic anhydride, butyric anhydride, valeric anhydride, caproic anhydride, enanthic anhydride, caprylic anhydride, pelargonic anhydride, capric anhydride, lauric anhydride, myristic anhydride, palmitic anhydride, stearic anhydride, oleic anhydride, linoleic anhydride, linolenic anhydride, benzoic anhydride, phthalic anhydride, maleic anhydride, and succinic anhydride. Among these, acetic anhydride is particularly preferred.

The allyl alcohol is not particularly limited, and examples thereof include methallyl alcohol, acryl alcohol, 2-hydroxymethyl-1-butene, and α-hydroxymethylstyrene.

### Ionic Liquid

Next, an ionic liquid will be described. The ionic liquid is a solvent for dissolving and/or dispersing a raw material (e.g., a polysaccharide) (particularly, a solvent for dissolving a raw material). Specific examples of the ionic liquid will be described later in the section of Ionic Liquid. Therefore, specific examples of the ionic liquid are omitted herein. As the ionic liquid, each specific ionic liquid exemplified in the section of "Ionic Liquid" can be used.

### Solvent Other Than Ionic Liquid [Solvent (S)]

Next, a solvent other than the ionic liquid will be described. Herein, in the present specification, the solvent may be referred to as a "solvent (S)." The solvent (S) is a solvent capable of further exhibiting the function of dissolving and/or dispersing the raw material when used together with the ionic liquid, and is also referred to as a cosolvent for the ionic liquid.

The solvent (S) is not particularly limited, and may be appropriately selected in consideration of miscibility with the ionic liquid. Moreover, when the raw material is a polysaccharide, that is, when the mixed liquid (B) is a mixed liquid obtained when the polysaccharide is esterified, the solvent (S) can be appropriately selected in consideration of the affinity with the polysaccharide and the esterified product of the polysaccharide, the viscosity of the mixture of the polysaccharide and the ionic liquid, and the like. Furthermore, the solvent (S) preferably does not react with the ionic liquid and, when being mixed with the ionic liquid, has high solubility of the polysaccharide and the esterified product of the polysaccharide. One type of the solvent (S) may be used, or two or more types of the solvents (S) may be used.

Specific examples of the solvent (S) include nitriles such as acetonitrile; sulfoxides such as dimethyl sulfoxide (DMSO); sulfones such as cyclic sulfone (e.g., sulfolane); ethers such as cyclic ether (e.g., 1,3-dioxolane, 1,4-dioxane, and tetrahydrofuran); amides such as N,N-dimethylformamide (DMF) and N,N-dimethylacetamide (DMAc); lactams such as N-methyl-2-pyrrolidone (NMP); lactones such as γ-butyrolactone; and amines such as pyridine. Among them, the solvent (S) is preferably at least one type selected from the group consisting of sulfoxides, sulfones, amides, and lactams, more preferably at least one type selected from the group consisting of dimethyl sulfoxide (DMSO), sulfolane, N,N-dimethylformamide (DMF), and N-methyl-2-pyrrolidone (NMP), even more preferably at least one type selected from the group consisting of N,N-dimethylformamide (DMF) and N-methyl-2-pyrrolidone (NMP), and particularly preferably N-methyl-2-pyrrolidone (NMP) from the viewpoint of miscibility with the ionic liquid and solubility of the esterified product of the polysaccharide.

As the solvent (S), an organic solvent to be blended in the mixed liquid (B) can also be used. When an organic solvent is used as the solvent (S), the extraction operation can be carried out without newly blending (adding) an organic solvent in the resulting mixed liquid (B1), which is preferable from the viewpoint that complicated operations need not be performed.

Above is the detailed description of the raw material, the esterifying agent, the ionic liquid, and the solvent other than the ionic liquid in a case where the mixed liquid (B) is the mixed liquid (B 1).

### Extraction

The method of the present disclosure is characterized in that the ionic liquid and the organic acid are separated by extraction from the mixed liquid (B) containing the ionic liquid and the organic acid. Specifically, water and/or an organic solvent (excluding an organic acid) is added to the mixed liquid (B) while the mixed liquid (B) is brought into contact with the water and the organic solvent, to prepare the mixed liquid (A), and the ionic liquid and the organic acid are separated by the resulting mixed liquid (A). This series of operations is referred to as extraction. Herein, hereinafter, the organic solvent (excluding an organic acid) is also referred to as an organic solvent (Or) as described later. The extraction operation of the present disclosure is a liquid-liquid extraction.

That is, the method of the present disclosure is characterized in that water and/or the organic solvent (Or) is blended in the mixed liquid (B) containing at least the ionic liquid and the organic acid during extraction. The method for blending water and/or the organic solvent (Or) in the mixed liquid (B), that is, the method for preparing the mixed liquid (A) is not particularly limited. Examples of the method for preparing the mixed liquid (A) include:
(1) a method for preparing the mixed liquid (A) by adding the organic solvent (Or) to the mixed liquid (B) containing the ionic liquid, the organic acid, and water;
(2) a method for preparing the mixed liquid (A) by adding water and the organic solvent (Or) to the mixed liquid (B) containing the ionic liquid, the organic acid, and water;
(3) a method for preparing the mixed liquid (A) by adding water to the mixed liquid (B) containing the ionic liquid, the organic acid, and the organic solvent (Or);
(4) a method for preparing the mixed liquid (A) by adding water and the organic solvent (Or) to the mixed liquid (B) containing the ionic liquid, the organic acid, and the organic solvent (Or);
(5) a method for preparing the mixed liquid (A) by adding water and the organic solvent (Or) to the mixed liquid (B) containing the ionic liquid and the organic acid;
(6) a method for preparing the mixed liquid (A) by adding water to a mixed liquid containing the ionic liquid, the organic acid, water, and the organic solvent (Or);
(7) a method for preparing the mixed liquid (A) by adding the organic solvent (Or) to a mixed liquid containing the ionic liquid, the organic acid, water, and the organic solvent (Or); and
(8) a method for preparing the mixed liquid (A) by adding water and the organic solvent (Or) to a mixed liquid containing the ionic liquid, the organic acid, water, and the organic solvent (Or). Water and/or the organic solvent (Or) may be blended (added) after the ionic liquid and the organic acid have been mixed, or may be collectively mixed with the ionic liquid and the organic acid.

When the mixed liquid (B) is the mixed liquid (B1), water and/or the organic solvent (Or) is blended (added) in a pre-esterification reaction system of a specific raw material to perform various reactions, and consequently at least one type selected from the group consisting of water and the organic solvent (Or) may be blended in the resulting mixed liquid. Alternatively, at least one type selected from the group consisting of water and the organic solvent (Or) may be blended (added) in the post-reaction mixed liquid (i.e., the mixed liquid (B1)) for the preparation.

In the method of the present disclosure, the extraction operation may be carried out for the mixed liquid (B) as it is (i.e., without pretreatment), or the extraction operation may be carried out for the mixed liquid (B) after pretreatment. Examples of such pretreatment include treatment with a strong acid such as sulfuric acid, hydrochloric acid, or nitric acid. It is also considered that, when the pretreatment with a strong acid is performed, the ionic liquid in the mixed liquid (B) forms a strong acid salt, and the amount of the ionic liquid that moves to the aqueous phase side increases by the subsequent extraction. However, the method of the present disclosure exerts the effects that, since the mixed liquid (B) contains a specific component, the organic acid can be separated by extraction, and as a result, the ionic liquid can be easily and efficiently recovered. That is, the method of the present disclosure exerts the advantageous effects without the pretreatment. Therefore, the mixed liquid (B) is preferably subjected to the extraction operation as it is (i.e., without the pretreatment). In other words, the structural formula of the ionic liquid recovered by the method of the present disclosure is preferably the same as the structural formula of the ionic liquid (in the mixed liquid (B)) before the extraction.

The extraction of the mixed liquid (B) allows separation of the ionic liquid and the organic acid. Alternatively, the ionic liquid and the organic acid may be separated by blending water and/or the organic solvent (Or) in the mixed liquid (B) followed by extraction. Herein, it is preferable that the organic acid is extracted on the organic solvent phase side (oil phase) and the ionic liquid is extracted on the aqueous phase side. Accordingly, the ionic liquid is recovered in a form separated from the organic acid. Specifically, the mixture of the ionic liquid and water is subjected to, for example, distillation, and thereby the ionic liquid is finally recovered.

The operation (extraction operation) for recovering the ionic liquid by extraction of the mixed liquid (B) can be performed in an apparatus that brings an aqueous phase and an oil phase (organic solvent phase) into contact with each other. As such an apparatus, for example, an extraction apparatus of a mixer-settler type extraction column, a perforated plate type, a packed column type, a baffle column type, a vibrating perforated plate type, a stirring mixing type, a pulsating packing type, a centrifugal extraction type, or the like can be used.

The temperature for extracting the mixed liquid (B) is not particularly limited, and is, for example, preferably from 0 to 100°C, more preferably from 1 to 90°C, even more preferably from 3 to 80°C, and still more preferably from 5 to 70°C.

The pressure for extracting the mixed liquid (B) is not particularly limited, and is, for example, preferably from 10 to 2000 hPa, more preferably from 100 to 1500 hPa, and even more preferably from 500 to 1200 hPa (e.g., atmospheric pressure).

The ionic liquid can be separated from the mixed liquid (B) by the extraction operation. In general, an aqueous phase, which mainly contains the ionic liquid and water, and an oil phase, which mainly contains the organic acid and the organic solvent, are obtained by the extraction operation. The ionic liquid can be separated from the aqueous phase containing the ionic liquid and water by a known or commonly used purification method such as evaporation of water. That is, according to the method of the present disclosure, a high-purity ionic liquid can be obtained by extraction which is an easy method.

### Ionic Liquid

Next, an ionic liquid will be described. The ionic liquid is a liquid component separated from the organic acid by the extraction operation (extraction step). Moreover, when the mixed liquid (B) is the mixed liquid (B1), the ionic liquid is also a solvent for dissolving and/or dispersing a raw material (e.g., a polysaccharide) (particularly, a solvent for dissolving a raw material). The ionic liquid of the present disclosure is water-soluble.

The ionic liquid of the present disclosure is not particularly limited as long as the ionic liquid is a water-soluble ionic liquid. The ionic liquid may be, for example, at least one water-soluble ionic liquid selected from the group consisting of an acidic ionic liquid, a neutral ionic liquid, and a basic ionic liquid. Note that, in the present specification, the "ionic liquid" is a salt composed of a cation component and an anion component, and the liquid property thereof can be specified by the acid dissociation constant (pKa, calculated value in vacuum) of the conjugate acid of the anion. For example, a basic ionic liquid has an acid dissociation constant of 2 to 19. One type of the ionic liquid may be used alone, or two or more types of the ionic liquids may be used.

### Cation Component

Examples of the cation component in the ionic liquid include an imidazolium cation, a pyridinium cation, a pyrrolidinium cation, a piperidinium cation, a quaternary ammonium cation, and a quaternary phosphonium cation.

Examples of the imidazolium cation include a cation represented by the following Formula (1). The cation represented by Formula (1) includes a tautomer thereof and a cation represented by a structural formula having a resonance relationship with Formula (1).

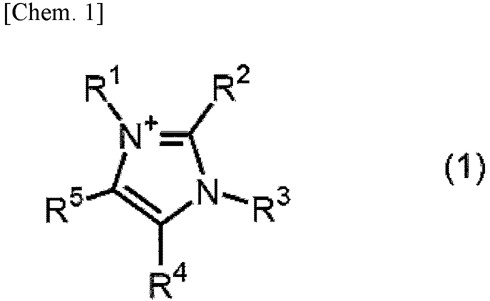

In Formula (1), R¹ and R³ are, identically or differently, substituted or unsubstituted alkyl groups, alkenyl groups, alkoxyalkyl groups, or substituted or unsubstituted phenyl groups, and R², R⁴, and R⁵ are, identically or differently, hydrogen atoms, substituted or unsubstituted alkyl groups, alkenyl groups, alkoxyalkyl groups, or substituted or unsubstituted phenyl groups.

Examples of the substituted or unsubstituted alkyl groups in R¹ to R⁵ include linear or branched alkyl groups having 1 to 20 (preferably 1 to 10, more preferably 2 to 6, and even more preferably 2 to 4) carbon atoms, such as a methyl group, an ethyl group, a n-propyl group, an isopropyl group, a butyl group, a hexyl group, and an octyl group. A sulfo group may be attached to the ends of these alkyl groups. Moreover, examples of the alkenyl groups include linear or branched alkenyl groups having 2 to 20 (preferably 2 to 10, more preferably 2 to 6, and even more preferably 2 to 4) carbon atoms, such as a vinyl group, a 1-propenyl group, a 2-propenyl group, a 1-butenyl group, a 2-butenyl group, a 1-pentenyl group, a 2-pentenyl group, a 1-hexenyl group, a 2-hexenyl group, and a 1-octenyl group. Furthermore, examples of the alkoxyalkyl groups include linear or branched alkoxyalkyl groups having 2 to 20 (preferably 2 to 10, more preferably 2 to 6, and even more preferably 2 to 4) carbon atoms, such as a methoxymethyl group, an ethoxymethyl group, a 1-methoxyethyl group, a 2-methoxyethyl group, a 1-ethoxyethyl group, and a 2-ethoxyethyl group. Further, examples of the substituted or unsubstituted phenyl groups include a phenyl group that may be substituted with 1 to 2 groups selected from a hydroxyl group, a halogen atom, a lower alkoxy group, a lower alkenyl group, a methylsulfonyloxy group, a substituted or unsubstituted lower alkyl group, a substituted or unsubstituted amino group, a substituted or unsubstituted phenyl group, a substituted or unsubstituted phenoxy group, or a substituted or unsubstituted pyridyl group.

R¹ and R³ are preferably alkyl groups, alkenyl groups, or substituted or unsubstituted phenyl groups, and more preferably linear alkyl groups having 1 to 6 carbon atoms. Moreover, it is particularly preferable that one of R¹ and R³ is a linear alkyl group having 1 to 4 carbon atoms, the other is a linear alkyl group having 2 to 6 carbon atoms, and these alkyl groups have different numbers of carbon atoms. R², R⁴, and R⁵ are preferably hydrogen atoms, alkyl groups, alkenyl groups, or substituted or unsubstituted phenyl groups, and more preferably hydrogen atoms or alkyl groups.

Examples of the imidazolium cation include cations of imidazoliums, such as 1,3-dimethylimidazolium, 1-ethyl-3-methylimidazolium, 1-butyl-3-methylimidazolium, 1-hexyl-3-methylimidazolium, 1-octyl-3-methylimidazolium, 1-decyl-3-methylimidazolium, 1-tetradecyl-3-methylimidazolium, 1-hexadecyl-3-methylimidazolium, 1-octadecyl-3-methylimidazolium, 1-allyl-3-methylimidazolium, 1-ethyl-2,3-dimethylimidazolium, 1-butyl-2,3-dimethylimidazolium, and 1-hexyl-2,3-dimethylimidazolium. Among them, a cation of 1-ethyl-3-methylimidazolium (Emim⁺) is particularly preferred.

Examples of the pyridinium cation include a cation represented by the following Formula (2). The cation represented by Formula (2) includes a tautomer thereof and a cation represented by a structural formula having a resonance relationship with Formula (2).

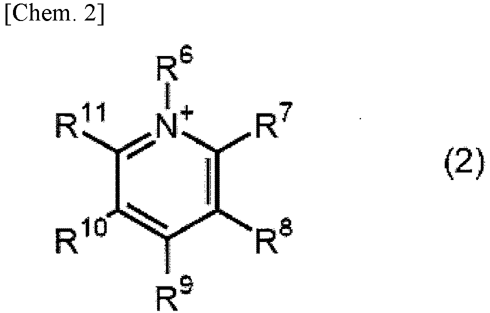

In Formula (2), R⁶ is an alkyl group, an alkenyl group, an alkoxyalkyl group, or a substituted or unsubstituted phenyl group, and R⁷ to R¹¹ are, identically or differently, hydrogen atoms, alkyl groups, alkenyl groups, alkoxyalkyl groups, or substituted or unsubstituted phenyl groups.

Examples of the alkyl groups, the alkenyl groups, the alkoxyalkyl groups, and the substituted or unsubstituted phenyl groups in R⁶ to R¹¹ are the same as those described as R¹ to R⁵ of the cation represented by Formula (1).

R⁶ is preferably an alkyl group and more preferably a linear alkyl group having 1 to 6 carbon atoms. R⁶ to R¹¹ are preferably hydrogen atoms or alkyl groups and more preferably hydrogen atoms.

Examples of the pyridinium cation include cations of pyridiniums, such as 1-ethylpyridinium, 1-butylpyridinium, 1-hexylpyridinium, 1-butyl-4-methylpyridinium, 1-butyl-3-methylpyridinium, 1-hexyl-4-methylpyridinium, 1-hexyl-3-methylpyridinium, 1-octyl-4-methylpyridinium, 1-octyl-3-methylpyridinium, 1-butyl-3,4-dimethylpyridinium, and 1-butyl-3,5-dimethylpyridinium. Among them, a cation of 1-octyl-4-methylpyridinium is particularly preferred.

Examples of the pyrrolidinium cation include a cation represented by the following Formula (3). The cation represented by Formula (3) includes a tautomer thereof.

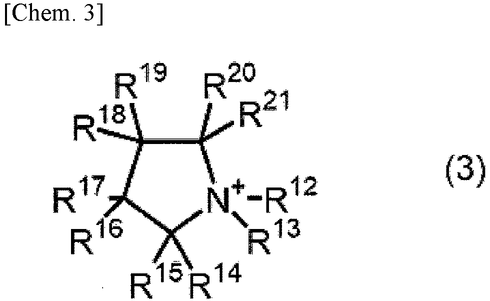

In Formula (3), R¹² and R¹³ are, identically or differently, alkyl groups, alkenyl groups, alkoxyalkyl groups, or substituted or unsubstituted phenyl groups, and R¹⁴ to R²¹ are, identically or differently, hydrogen atoms, alkyl groups, alkenyl groups, alkoxyalkyl groups, or substituted or unsubstituted phenyl groups.

Examples of the alkyl groups, the alkenyl groups, the alkoxyalkyl groups, and the substituted or unsubstituted phenyl groups in R¹² to R²¹ are the same as those described as R¹ to R⁵ of the cation represented by Formula (1).

R¹² and R¹³ are preferably alkyl groups and more preferably linear alkyl groups having 1 to 6 carbon atoms. R¹⁴ to R²¹ are preferably hydrogen atoms or alkyl groups and more preferably hydrogen atoms.

The pyrrolidinium cation is particularly preferably a cation of 1-butyl-1-methylpyrrolidinium.

Examples of the piperidinium cation include a cation represented by the following Formula (4). The cation represented by Formula (4) includes a tautomer thereof.

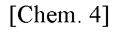

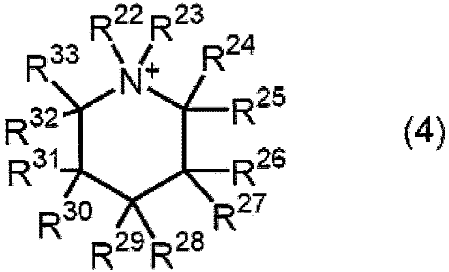

In Formula (4), R²² and R²³ are, identically or differently, alkyl groups, alkenyl groups, alkoxyalkyl groups, or substituted or unsubstituted phenyl groups, and R²⁴ to R³³ are, identically or differently, hydrogen atoms, alkyl groups, alkenyl groups, alkoxyalkyl groups, or substituted or unsubstituted phenyl groups.

Examples of the alkyl groups, the alkenyl groups, the alkoxyalkyl groups, and the substituted or unsubstituted phenyl groups in R²² to R³³ are the same as those described as R¹ to R⁵ of the cation represented by Formula (1).

R²² and R²³ are preferably alkyl groups and more preferably linear alkyl groups having 1 to 6 carbon atoms. R²⁴ to R³³ are preferably hydrogen atoms or alkyl groups and more preferably hydrogen atoms.

The piperidinium cation is preferably a cation of 1-butyl-1-methylpiperidinium.

Examples of the quaternary ammonium cation include an ammonium cation represented by the following Formula (5). The cation represented by Formula (5) includes a tautomer thereof.

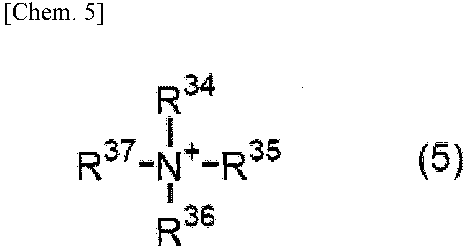

In Formula (5), R³⁴ to R³⁷ are, identically or differently, alkyl groups, alkenyl groups, alkoxyalkyl groups, or substituted or unsubstituted phenyl groups.

Examples of the alkyl groups in R³⁴ to R³⁷ include linear or branched alkyl groups having 1 to 20 (preferably 1 to 10, more preferably 2 to 6, and even more preferably 2 to 4) carbon atoms, such as a methyl group, an ethyl group, a n-propyl group, an isopropyl group, a butyl group, a hexyl group, and an octyl group. Moreover, examples of the alkenyl groups include linear or branched alkenyl groups having 2 to 20 (preferably 2 to 10, more preferably 2 to 6, and even more preferably 2 to 4) carbon atoms, such as a vinyl group, a 1-propenyl group, a 2-propenyl group, a 1-butenyl group, a 2-butenyl group, a 1-pentenyl group, a 2-pentenyl group, a 1-hexenyl group, a 2-hexenyl group, and a 1-octenyl group. Furthermore, examples of the alkoxyalkyl groups include linear or branched alkoxyalkyl groups having 2 to 20 (preferably 2 to 10, more preferably 2 to 6, and even more preferably 2 to 4) carbon atoms, such as a methoxymethyl group, an ethoxymethyl group, a 1-methoxyethyl group, a 2-methoxyethyl group, a 1-ethoxyethyl group, and a 2-ethoxyethyl group. Further, examples of the substituted or unsubstituted phenyl groups include a phenyl group that may be substituted with 1 to 2 groups selected from a hydroxyl group, a halogen atom, a lower alkoxy group, a lower alkenyl group, a methylsulfonyloxy group, a substituted or unsubstituted lower alkyl group, a substituted or unsubstituted amino group, a substituted or unsubstituted phenyl group, a substituted or unsubstituted phenoxy group, or a substituted or unsubstituted pyridyl group.

R³⁴ to R³⁷ are preferably alkyl groups and more preferably linear alkyl groups having 1 to 6 carbon atoms.

Examples of the quaternary ammonium cation include cations of ammoniums, such as trimethylpropylammonium, trimethylbutylammonium, triethylmethylammonium, trioctylmethylammonium, tetramethylammonium, tetraethylammonium, tetrabutylammonium, tetrapentylammonium, and tetrahexylammonium.

Examples of the quaternary phosphonium cation include a phosphonium cation represented by the following Formula (6). The cation represented by Formula (6) includes a tautomer thereof.

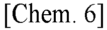

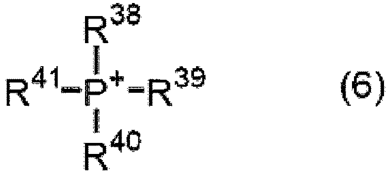

In Formula (6), R³⁸ to R⁴¹ are, identically or differently, alkyl groups, alkenyl groups, alkoxyalkyl groups, or substituted or unsubstituted phenyl groups.

Examples of the alkyl groups in R³⁸ to R⁴¹ include linear or branched alkyl groups having 1 to 20 (preferably 1 to 10, more preferably 2 to 6, and even more preferably 2 to 4) carbon atoms, such as a methyl group, an ethyl group, a n-propyl group, an isopropyl group, a butyl group, a hexyl group, and an octyl group. Moreover, examples of the alkenyl groups include linear or branched alkenyl groups having 2 to 20 (preferably 2 to 10, more preferably 2 to 6, and even more preferably 2 to 4) carbon atoms, such as a vinyl group, a 1-propenyl group, a 2-propenyl group, a 1-butenyl group, a 2-butenyl group, a 1-pentenyl group, a 2-pentenyl group, a 1-hexenyl group, a 2-hexenyl group, and a 1-octenyl group. Furthermore, examples of the alkoxyalkyl groups include linear or branched alkoxyalkyl groups having 2 to 20 (preferably 2 to 10, more preferably 2 to 6, and even more preferably 2 to 4) carbon atoms, such as a methoxymethyl group, an ethoxymethyl group, a 1-methoxyethyl group, a 2-methoxyethyl group, a 1-ethoxyethyl group, and a 2-ethoxyethyl group. Further, examples of the substituted or unsubstituted phenyl groups include a phenyl group that may be substituted with 1 to 2 groups selected from a hydroxyl group, a halogen atom, a lower alkoxy group, a lower alkenyl group, a methylsulfonyloxy group, a substituted or unsubstituted lower alkyl group, a substituted or unsubstituted amino group, a substituted or unsubstituted phenyl group, a substituted or unsubstituted phenoxy group, or a substituted or unsubstituted pyridyl group.

Examples of the quaternary phosphonium cation include cations of quaternary phosphoniums, such as tetramethylphosphonium, tetraethylphosphonium, tetrabutylphosphonium, tetrahexylphosphonium, tetraoctylphosphonium, triethylmethylphosphonium, and tributylethylphosphonium.

### Anion Component

Examples of an anion component in the ionic liquid of the present disclosure include a halogen anion, a pseudohalogen anion, a carboxylate anion, a phosphate-based anion, an amino acid anion, phenolate, pyrimidinolate, a tetrafluoroborate ion (BF⁴⁻), a sulfomethyl ion (CH₃SO₃⁻), methyl phosphonate, a sulfate ion, and PF₆⁻.

The halogen anion is not particularly limited, and examples thereof include a fluoride ion (F⁻), a chloride ion (Cl⁻), an iodide ion (I⁻), and a bromide ion (Br⁻).

The pseudohalogen anion is not particularly limited, and examples thereof include a cyan anion, a thiocyanate anion, a cyanate anion, a fulminate anion, and an azide anion.

The carboxylate anion is not particularly limited, and examples thereof include a monocarboxylate anion or a dicarboxylate anion having 1 to 18 carbons. Examples of the carboxylate anion include a formate anion, an acetate anion, a propionate anion, a butyrate anion, a valerate anion, a fumarate anion, an oxalate anion, a lactate anion, and a pyruvate anion. Note that the anion component of the ionic liquid preferably contains a carboxylate anion (OAc⁻) from the viewpoint that the ionic liquid can be easily and efficiently recovered.

The phosphate-based anion is not particularly limited, and examples thereof include a phosphate anion and a phosphate ester anion having 1 to 40 carbons. Examples of the phosphate ester anion include a methyl phosphate monoester anion, an octyl phosphate monoester anion, an octyl phosphate diester anion, a lauryl phosphate monoester anion, a lauryl phosphate diester anion, a stearyl phosphate monoester anion, a stearyl phosphate diester anion, an eicosyl phosphate monoester anion, and an eicosyl phosphate diester anion.

Commercially available products of the above ionic liquids can be used. Moreover, the ionic liquid can also be produced by a known technique.

The ionic liquid is an arbitrary combination of the cation component and the anion component. For example, an ionic liquid containing an imidazolium cation or a quaternary ammonium cation as a cation component is preferred. In particular, the reason that the imidazolium cation is preferred is considered to be due to the skeleton of Formula (1) in terms of affinity and separation efficiency in the extraction operation. Moreover, an ionic liquid containing a carboxylate anion as an anion component is preferred. More specifically, the ionic liquid is more preferably at least one type selected from the group consisting of 1-ethyl-3-methylimidazolium acetate (EmimOAc), 1-ethyl-2,3-dimethylimidazolium acetate (EDmimOAc), 1-butyl-3-methylimidazolium acetate, 1-hexyl-3-methylimidazolium acetate, and tetrabutylammonium acetate. Furthermore, the ionic liquid is even more preferably at least one type selected from the group consisting of 1-ethyl-3-methylimidazolium acetate (EmimOAc) and 1-ethyl-2,3-dimethylimidazolium acetate (EDmimOAc), and particularly preferably 1-ethyl-3-methylimidazolium acetate (EmimOAc).

### Organic Acid

Next, the organic acid will be described. The organic acid is a component separated from the ionic liquid by the extraction operation.

Examples of the organic acid include carboxylic acids (preferably C₁₋₁₈ carboxylic acid, more preferably C₁₋₁₀ carboxylic acid, even more preferably C₁₋₆ carboxylic acid, and particularly preferably C₂₋₄ carboxylic acid) such as formic acid, acetic acid, propionic acid, and trifluoroacetic acid. Moreover, the carboxylic acid is preferably a C₉₋₁₈ carboxylic acid and is also preferably a C₁₋₇ carboxylic acid. One type of the organic acid may be used, or two or more types of the organic acids may be used.

The organic acid may be derived from the esterifying agent. Examples of the esterifying agent in this case include acid anhydride. For example, when the esterifying agent used for esterifying the polysaccharide is acid anhydride, the resulting mixed liquid (B2) may contain the acid anhydride and/or an organic acid derived from the acid anhydride. The method of the present disclosure can be suitably used when an ionic liquid is recovered from the mixed liquid (B2).

When the organic acid is carboxylic acid, a C₁₋₆ carboxylic acid content relative to the carboxylic acid (total amount) is, for example, preferably 70 wt.% or more, more preferably 80 wt.% or more, even more preferably 90 wt.% or more, still more preferably 95 wt.% or more, and particularly preferably 99 wt.% or more. In this case, the upper limit of the C₁₋₆ carboxylic acid content relative to the carboxylic acid (total amount) is preferably, for example, 100 wt.%.

The molar ratio of the ionic liquid to the organic acid (ionic liquid:organic acid) contained in the mixed liquid (A) is not particularly limited, and is, for example, preferably 1:100 to 100:1, more preferably 10:90 to 90:10, even more preferably 20:80 to 80:20, and particularly preferably 30:70 to 70:30. When the ratio of the ionic liquid to the organic acid contained in the mixed liquid is within the above range, there is a tendency that the ionic liquid can be easily and efficiently recovered from the mixed liquid. Note that, when the mixed liquid (A) is prepared by blending the solvent in the mixed liquid (B), the molar ratio of the ionic liquid to the organic acid contained in the mixed liquid (B) is also preferably in the above range.

### Water

Next, water will be described. Water may be already contained in the mixed liquid (B), may be newly blended in the mixed liquid (B), or may be already contained in the mixed liquid (B) and further blended in the mixed liquid (B). Water constitutes an aqueous phase in the mixed liquid (A) by contacting with an organic solvent (excluding an organic acid) described later.

The water of the present disclosure is not particularly limited, and, for example, industrial water, tap water, ion-exchanged water, purified water, distilled water, or the like can be used.

The amount of water used in the method of the present disclosure is not particularly limited, and is, for example, preferably 0.01 mol or more, more preferably 0.1 mol or more, even more preferably 1 mol or more, even more preferably 5 mol or more, even more preferably 10 mol or more, even more preferably 15 mol or more, even more preferably 20 mol or more, and even more preferably 25 mol or more relative to 1 mol of the ionic liquid. Moreover, the amount of water is, for example, preferably 1000 mol or less, more preferably 500 mol or less, even more preferably 300 mol or less, even more preferably 250 mol or less, even more preferably 200 mol or less, even more preferably 150 mol or less, even more preferably 100 mol or less, even more preferably 75 mol or less, even more preferably 50 mol or less, and even more preferably 40 mol or less relative to 1 mol of the ionic liquid. Furthermore, the amount of water is preferably from 0.01 to 1000 mol, more preferably from 0.1 to 500 mol, even more preferably 1 to 300 mol, and preferably 25 to 40 mol relative to 1 mol of the ionic liquid. When the amount of water contained in the mixed liquid (A) is within the above range, the ratio of the ionic liquid contained in the aqueous phase is high, and as a result, there is a tendency that the ionic liquid can be easily and efficiently recovered from the mixed liquid (A) and/or the mixed liquid (B).

### Organic Solvent (excluding organic acid) [Organic Solvent (Or)]

Next, the organic solvent (excluding an organic acid) will be described. Herein, in the present specification, the organic solvent may be referred to as an "organic solvent (Or)." The organic solvent (Or) may be already contained in the mixed liquid (B), may be newly blended in the mixed liquid (B), or may be already contained in the mixed liquid (B) and further blended in the mixed liquid (B). The organic solvent (Or) constitutes an oil phase in the mixed liquid (A) by contacting with the aforementioned water.

The organic solvent (Or) is not particularly limited as long as the organic solvent (Or) excludes the above-described organic acid and forms an oil phase (organic solvent phase) when mixed with water. Examples thereof include at least one type selected from the group consisting of ethers, ketones, esters, aryls, alkanes, and cycloalkanes. One type of the organic solvent (Or) can be used, or two or more types of the organic solvents (Or) can be used in combination.

The ethers are not particularly limited, and examples thereof include dialkyl ethers, such as diethyl ether, diisopropyl ether, and methyl tert-butyl ether, and dioxane. The ketones are not particularly limited, and examples thereof include methyl ethyl ketone, methyl isobutyl ketone, cyclohexanone, and acetophenone. The esters are not particularly limited, and examples thereof include methyl acetate, ethyl acetate, butyl acetate, and isopropyl acetate. The aryls are not particularly limited, and examples thereof include benzene, toluene, xylene, methoxybenzene, and ethylbenzene. The alkanes are not particularly limited, and examples thereof include n-hexane and n-heptane. The cycloalkanes are not particularly limited, and examples thereof include cyclohexane.

The organic solvent (Or) is preferably at least one type selected from the group consisting of ethers, ketones, and esters, more preferably at least one type selected from the group consisting of ethers (particularly dialkyl ethers) and esters, even more preferably at least one type selected from the group consisting of diethyl ethers, diisopropyl ether, and ethyl acetate, and particularly preferably ethyl acetate, from the viewpoint that the ionic liquid can be easily and efficiently recovered from the mixed liquid (A).

In the method of the present disclosure, the amount of the organic solvent (Or) is not particularly limited, and is, for example, preferably 1 part by weight or more, more preferably 10 parts by weight or more, even more preferably 20 parts by weight or more, even more preferably 30 parts by weight or more, and even more preferably 50 parts by weight or more relative to 100 parts by weight of the total amount of the ionic liquid, the organic acid, and the water contained in the mixed liquid (A). Moreover, the amount of the organic solvent (Or) is not particularly limited, and is, for example, preferably 10000 parts by weight or less, more preferably 6000 parts by weight or less, even more preferably 5000 parts by weight or less, even more preferably 4000 parts by weight or less, and even more preferably 2000 parts by weight or less relative to 100 parts by weight of the total amount of the ionic liquid, the organic acid, and the water contained in the mixed liquid (A). The upper limit and the lower limit of the amount of the organic solvent (Or) are preferably from 1 to 10000 parts by weight, more preferably from 10 to 6000 parts by weight, even more preferably from 20 to 5000 parts by weight, even more preferably 30 to 4000 parts by weight, and even more preferably 50 to 2000 parts by weight relative to 100 parts by weight of the total amount of the ionic liquid, the organic acid, and the water contained in the mixed liquid (A). When the amount of the organic solvent (Or) blended in the mixed liquid (A) is within the above range, the ratio of the organic acid contained in the oil phase is high, and as a result, there is a tendency that the ionic liquid can be easily and efficiently recovered from the mixed liquid (A).

### Other Solvent [Solvent (T)]

In the method of the present disclosure, the mixed liquid (A) may further contain a solvent in addition to the ionic liquid, the organic acid, the water, and the organic solvent (Or). Hereinafter, such a solvent is referred to as an "other solvent" and is also referred to as a "solvent (T)." The solvent (T) may be already contained in the mixed liquid (B), may be newly blended in the mixed liquid (B), or may be already contained in the mixed liquid (B) and further blended in the mixed liquid (B). That is, when the mixed liquid (A) contains the solvent (T), the process of containing the solvent (T) may be any of the processes described above. When the solvent (T) is already contained from the beginning at the time of the mixed liquid (B), the solvent (T) may be contained as the solvent (S). That is, when the mixed liquid (B) is the mixed liquid (B1), the solvent (S) serves as the solvent (T) as it is when the mixed liquid (A) is obtained.

Specific examples of the solvent (T) include the solvents described as the "solvent other than the ionic liquid [solvent (S)]" that can be contained in the mixed liquid (B).

Among the solvents (T), at least one type selected from the group consisting of sulfoxides, sulfones, amides, and lactams is preferred from the viewpoint of miscibility with the ionic liquid and the viewpoint of solubility of the esterified product of the polysaccharide. Among them, when at least one type selected from the group consisting of dimethyl sulfoxide (DMSO), sulfolane, N,N-dimethylformamide (DMF), and N-methyl-2-pyrrolidone (NMP) is used in separating the organic acid, the distribution ratio of the ionic liquid to the aqueous phase side tends to increase. This tendency becomes more remarkable by using at least one type selected from the group consisting of N,N-dimethylformamide (DMF) and N-methyl-2-pyrrolidone (NMP), and becomes further remarkable by using N-methyl-2-pyrrolidone (NMP).

The content of the solvent (T) contained in the mixed liquid (A) is not particularly limited, and is, for example, preferably 0.01 wt.% or more, more preferably 0.05 wt.% or more, even more preferably 0.1 wt.% or more, still further preferably 0.3 wt.% or more, and particularly preferably 0.5 wt.% or more. Moreover, the content of the solvent (T) contained in the mixed liquid (A) is not particularly limited, and is, for example, preferably 30 wt.% or less, more preferably 20 wt.% or less, even more preferably 10 wt.% or less, still further preferably 5 wt.% or less, and particularly preferably 4 wt.% or less. Note that, when the solvent (T) is blended in the mixed liquid (B), the resulting mixed liquid (A) is preferably prepared to be in the above range.

The content of the solvent (T) contained in the mixed liquid (B) is not particularly limited, and is, for example, preferably 1 wt.% or more, more preferably 3 wt.% or more, even more preferably 5 wt.% or more, still further preferably 10 wt.% or more, and particularly preferably 15 wt.% or more. Moreover, the content of the solvent (T) contained in the mixed liquid (B) is not particularly limited, and is, for example, preferably 90 wt.% or less and more preferably 80 wt.% or less.

### Composition and Composition for Extraction

The present embodiment encompasses a composition containing a water-soluble ionic liquid, an organic acid, water, and an organic solvent (excluding the organic acid). The composition may further contain another solvent. According to this composition, an ionic liquid having higher purity can be obtained by extraction. The composition in the present embodiment is preferably for extraction. Hereinafter, the composition for extraction will be described.

A preferred aspect of the water-soluble ionic liquid contained in the composition for extraction is the same as the preferred aspect described in the aforementioned section of Ionic Liquid. A preferred aspect of the organic acid contained in the composition for extraction is the same as the preferred aspect described in the aforementioned section of Organic Acid. A preferred aspect of the water contained in the composition for extraction is the same as the preferred aspect described in the aforementioned section of Water. A preferred aspect of the organic solvent (excluding an organic acid) contained in the composition for extraction is the same as the preferred aspect described in the aforementioned section of Organic Solvent (excluding organic acid) [Organic Solvent (Or)]. A preferred aspect of the other solvent contained in the composition for extraction is the same as the preferred aspect described in the aforementioned section of Other Solvent [Solvent (T)]. Preferred extraction conditions of the composition for extraction are the same as the preferred aspects described in the aforementioned section of Extraction. In particular, the above-described mixed liquid (A) can be regarded as the composition for extraction, and the type and amount of each preferred component, extraction conditions, and the like can be referred. The composition for extraction is obtained by blending water and/or the organic solvent (Or) in the mixed liquid (B) containing the ionic liquid and the organic acid. Therefore, not only the mixed liquid (A) but also the mixed liquid (B) can be referred for the type and amount of each preferred component of the composition for extraction, distillation conditions, and the like.

The ionic liquid of the composition for extraction is preferably at least one type selected from the group consisting of 1-ethyl-3-methylimidazolium acetate (EmimOAc) and 1-ethyl-2,3-dimethylimidazolium acetate (EDmimOAc), and more preferably 1-ethyl-3-methylimidazolium acetate (EmimOAc). The organic acid of the composition for extraction is preferably acetic acid.

The organic solvent of the composition for extraction is preferably at least one type selected from the group consisting of ethers, ketones, and esters, and more preferably at least one type selected from the group consisting of ethers (particularly dialkyl ethers) and esters.

The other solvent is preferably at least one type selected from the group consisting of dimethyl sulfoxide, N,N-dimethylformamide, and N-methyl-2-pyrrolidone, more preferably at least one type selected from the group consisting of N,N-dimethylformamide and N-methyl-2-pyrrolidone, and particularly preferably N-methyl-2-pyrrolidone.

Each configuration, a combination of the configurations, and the like in each embodiment are an example, and various additions, omissions, substitutions, and other changes are possible as appropriate without departing from the spirit of the present disclosure. The present disclosure is not limited by the embodiments and is limited only by the claims.

### Examples

The present disclosure will next be described in more detail by way of Examples, but the present disclosure is not limited to the Examples.

The specifications of the NMR instrument used in Examples are as follows.
Product name: JNM-ECZ600R
Manufacturer: JEOL Ltd.

### Example 1

0.73 g of EmimOAc and 0.27 g of acetic acid were mixed, and 0.79 g of water was further added to prepare a mixed liquid. The molar ratio of EmimOAc:acetic acid:water in the mixed liquid was 1:1:10. Further, ethyl acetate was added in an amount of 10 times the weight of the mixed liquid, followed by stirring. After allowing to stand at room temperature for one hour, 1 ml of each of the aqueous phase and the ethyl acetate phase was sampled, the weights of the components were determined by NMR, and the results were as the following.
Distribution ratio of acetic acid (acetic acid concentration in ethyl acetate/acetic acid concentration in water) = 0.115
Distribution ratio of EmimOAc (EmimOAc concentration in ethyl acetate/EmimOAc concentration in water) = 0.00132

### Example 2

0.73 g of EmimOAc and 0.26 g of acetic acid were mixed, and 1.57 g of water was further added to prepare a mixed liquid. The molar ratio of EmimOAc:acetic acid:water in the mixed liquid was 1:1:20. Further, ethyl acetate was added in an amount of 10 times the weight of the mixed liquid, followed by stirring. After allowing to stand at room temperature for one hour, 1 ml of each of the aqueous phase and the ethyl acetate phase was sampled, the weights of the components were determined by NMR, and the results were as the following.
Distribution ratio of acetic acid (acetic acid concentration in ethyl acetate/acetic acid concentration in water) = 0.318
Distribution ratio of EmimOAc (EmimOAc concentration in ethyl acetate/EmimOAc concentration in water) = 0.00117

### Example 3

0.73 g of EmimOAc and 0.27 g of acetic acid were mixed, and 2.39 g of water was further added to prepare a mixed liquid. The molar ratio of EmimOAc:acetic acid:water in the mixed liquid was 1:1:30. Further, ethyl acetate was added in an amount of 10 times the weight of the mixed liquid, followed by stirring. After allowing to stand at room temperature for one hour, 1 ml of each of the aqueous phase and the ethyl acetate phase was sampled, the weights of the components were determined by NMR, and the results were as the following.
Distribution ratio of acetic acid = 0.369
Distribution ratio of EmimOAc = 0.00096

### Example 4

0.73 g of EmimOAc and 0.27 g of acetic acid were mixed, and 7.86 g of water was further added to prepare a mixed liquid. The molar ratio of EmimOAc:acetic acid:water in the mixed liquid was 1:1:100. Further, ethyl acetate was added in an amount of 10 times the weight of the mixed liquid, followed by stirring. After allowing to stand at room temperature for one hour, 1 ml of each of the aqueous phase and the ethyl acetate phase was sampled, the weights of the components were determined by NMR, and the results were as the following.
Distribution ratio of acetic acid (acetic acid concentration in ethyl acetate/acetic acid concentration in water) = 0.427
The distribution ratio of EmimOAc (EmimOAc concentration in ethyl acetate) could not be detected.

### Example 5

0.73 g of EmimOAc and 0.27 g of acetic acid were mixed, and 2.40 g of water was further added to prepare a mixed liquid. The molar ratio of EmimOAc:acetic acid:water in the mixed liquid was 1: 1:30. Further, diethyl ether was added in an amount of 10 times the weight of the mixed liquid, followed by stirring. After allowing to stand at room temperature for one hour, 1 ml of each of the aqueous phase and the diethyl ether phase was sampled, the weights of the components were determined by NMR, and the results were as the following.
Distribution ratio of acetic acid (acetic acid concentration in diethyl ether/acetic acid concentration in water) = 0.561
Distribution ratio of EmimOAc (EmimOAc concentration in diethyl ether/EmimOAc concentration in water) = 0.00335

### Example 6

0.73 g of EmimOAc and 0.27 g of acetic acid were mixed, and 2.40 g of water was further added to prepare a mixed liquid. The molar ratio of EmimOAc:acetic acid:water in the mixed liquid was 1: 1:30. Further, diisopropyl ether was added in an amount of 10 times the weight of the mixed liquid, followed by stirring. After allowing to stand at room temperature for one hour, 1 ml of each of the aqueous phase and the diisopropyl ether phase was sampled, the weights of the components were determined by NMR, and the results were as the following.
Distribution ratio of acetic acid (acetic acid concentration in diisopropyl ether/acetic acid concentration in water) = 0.172
Distribution ratio of EmimOAc (EmimOAc concentration in diisopropyl ether/EmimOAc concentration in water) = 0.00883

### Example 7

0.73 g of EmimOAc and 0.27 g of acetic acid were mixed, and 3.86 g of water was further added to prepare a mixed liquid. The molar ratio of EmimOAc:acetic acid:water in the mixed liquid was 1: 1:50. Further, ethyl acetate was added in an amount of 10 times the weight of the mixed liquid, followed by stirring. After allowing to stand at room temperature for one hour, 1 ml of each of the aqueous phase and the ethyl acetate phase was sampled, the weights of the components were determined by NMR, and the results were as the following.
Distribution ratio of acetic acid (acetic acid concentration in ethyl acetate/acetic acid concentration in water) = 0.401
Distribution ratio of EmimOAc (EmimOAc concentration in ethyl acetate/EmimOAc concentration in water) = 0.000935

### Example 8

0.73 g of EmimOAc and 0.27 g of acetic acid were mixed, and 1.57 g of water and 0.43 g of NMP were further added to prepare a mixed liquid. The molar ratio of EmimOAc:acetic acid:water:NMP in the mixed liquid was 1:1:20:1. Further, ethyl acetate was added in an amount of 10 times the weight of the mixed liquid, followed by stirring. After allowing to stand at room temperature for one hour, 1 ml of each of the aqueous phase and the ethyl acetate phase was sampled, the weights of the components were determined by NMR, and the results were as the following.
Distribution ratio of acetic acid (acetic acid concentration in ethyl acetate/acetic acid concentration in water) = 0.286
Distribution ratio of EmimOAc (EmimOAc concentration in ethyl acetate/EmimOAc concentration in water) = 0.000990
Distribution ratio of NMP (NMP concentration in ethyl acetate/NMP concentration in water) = 0.386

Therefore, it was proved that it is preferable to further blend an organic solvent such as NMP in the case where distribution of more ionic liquid described above to the aqueous phase side is desired when the organic acid is separated by blending water and an organic solvent (e.g., ethyl acetate) in a mixed liquid containing the ionic liquid (e.g., EmimOAc) and the organic acid (e.g., acetic acid) followed by extraction.

### Example 9

The structural formula of EmimOAc recovered from the aqueous phase in Example 3 has the same structural formula as EmimOAc before the extraction (before recovery).

### Example 10

After water was evaporated from the mixed liquid of EmimOAc and water recovered in Example 3, the resulting EmimOAc (1 g) was mixed with cellulose (0.18 g, trade name "Avicel PH-101" available from Sigma-Aldrich, number average degree of polymerization 105) and stirred to dissolve the cellulose. This phenomenon proved that the ionic liquid recovered by the method of the present disclosure can be reused.

### Comparative Example 1

0.75 g of EmimOAc and 0.27 g of acetic acid were mixed to prepare a mixed liquid. The molar ratio of EmimOAc:acetic acid in the mixed liquid was 1: 1. Further, ethyl acetate was added in an amount of 10 times the weight of the mixed liquid followed by stirring to separate the mixed liquid into two phases of an ethyl acetate phase (upper phase) and an acetic acid phase (lower phase). After allowing to stand at room temperature for one hour, 1 ml sampling was performed, and the weights of the components was determined by NMR. From the result, it was found that the ethyl acetate phase contained 46.3 wt.% of EmimOAc and 51.6 wt.% of acetic acid. It was also found that the acetic acid phase contained 53.7 wt.% of EmimOAc.

To summarize the above, configurations of the present invention and variations thereof will be described below.
[1] A method for recovering an ionic liquid from a mixed liquid (B) containing the ionic liquid and an organic acid, in which
   the ionic liquid is water-soluble, and
   the method includes blending at least one type selected from the group consisting of water and an organic solvent (excluding the organic acid) (also referred to as an organic solvent (Or)) in the mixed liquid (B), and separating the organic acid by extraction.
[2] The method for recovering an ionic liquid according to [1], in which the mixed liquid (B) is a mixed liquid (B1) containing a reaction product obtained when a specific raw material is esterified with an esterifying agent in the ionic liquid used as a solvent.
[3] The method for recovering an ionic liquid according to [2], in which the raw material is a polysaccharide (e.g., one or more type selected from the group consisting of cellulose, hemicellulose, xylan, mannan, glucomannan, glucuronoxylan, starch, amylose, amylopectin, glycogen, dextrin, pectin, chitin, chitosan, agarose, carrageenan, isolichenan, laminaran, lichenan, glucan, inulin, levan, fructan, galactan, arabinan, pentosan, alginic acid, pectic acid, protuberic acid, colominic acid, porphyran, fucoidan, ascophyllan, locust bean gum, guar gum, tamarind gum, tara gum, and gum arabic).
[4] The method for recovering an ionic liquid according to [2] or [3], in which the esterifying agent is one or more type selected from the group consisting of a chain ester compound, a cyclic ester compound, unsaturated aldehyde, saturated aldehyde, acid halide, acid anhydride, and allyl alcohol.
[5] The method for recovering an ionic liquid according to [4], in which the acid anhydride is one or more type selected from the group consisting of acetic anhydride, propionic anhydride, butyric anhydride, valeric anhydride, caproic anhydride, enanthic anhydride, caprylic anhydride, pelargonic anhydride, capric anhydride, lauric anhydride, myristic anhydride, palmitic anhydride, stearic anhydride, oleic anhydride, linoleic anhydride, linolenic anhydride, benzoic anhydride, phthalic anhydride, maleic anhydride, and succinic anhydride.
[6] The method for recovering an ionic liquid according to any one of [1] to [5], in which the mixed liquid (B) contains a solvent (solvent (S)) other than the ionic liquid.
[7] The method for recovering an ionic liquid according to [6], in which the solvent (S) is at least one type selected from the group consisting of nitriles such as acetonitrile; sulfoxides such as dimethyl sulfoxide (DMSO); sulfones such as a cyclic sulfone (e.g., sulfolane); ethers such as a cyclic ether (e.g., 1,3-dioxolane, 1 4-dioxane, and tetrahydrofuran); amides such as N,N-dimethylformamide (DMF) and N,N-dimethylacetamide (DMAc); lactams such as N-methyl-2-pyrrolidone (NMP); lactones such as γ-butyrolactone; and amines such as pyridine; at least one type selected from the group consisting of sulfoxides, sulfones, amides, and lactams; at least one type selected from the group consisting of dimethyl sulfoxide (DMSO), sulfolane, N,N-dimethylformamide (DMF), and N-methyl-2-pyrrolidone (NMP); at least one type selected from the group consisting of N,N-dimethylformamide (DMF) and N-methyl-2-pyrrolidone (NMP); or N-methyl-2-pyrrolidone (NMP).
[8] The method for recovering an ionic liquid according to any one of [1] to [7], in which a method for preparing a mixed liquid (A) containing the ionic liquid, the organic acid, the water, and the organic solvent (Or) by blending at least one type selected from the group consisting of the water and the organic solvent (Or) in the mixed liquid (B) is: a preparation method by adding the organic solvent (Or) to the mixed liquid (B) containing the ionic liquid, the organic acid, and the water; a preparation method by adding the water and the organic solvent (Or) to the mixed liquid (B) containing the ionic liquid, the organic acid, and the water; a preparation method by adding the water to the mixed liquid (B) containing the ionic liquid, the organic acid, and the organic solvent (Or); a preparation method by adding the water and the organic solvent (Or) to the mixed liquid (B) containing the ionic liquid, the organic acid, and the organic solvent (Or); a preparation method by adding the water and the organic solvent (Or) to the mixed liquid (B) containing the ionic liquid and the organic acid; a preparation method by adding the water to a mixed liquid containing the ionic liquid, the organic acid, the water, and the organic solvent (Or); a preparation method by adding the organic solvent (Or) to a mixed liquid containing the ionic liquid, the organic acid, the water, and the organic solvent (Or); or a preparation method by adding the water and the organic solvent (Or) to a mixed liquid containing the ionic liquid, the organic acid, the water, and the organic solvent (Or).
[9] The method for recovering an ionic liquid according to any one of [1] to [8], in which the extraction operation is performed without subjecting the mixed liquid (B) to pretreatment.
[10] The method for recovering an ionic liquid according to any one of [1] to [9], in which a structural formula of the recovered ionic liquid is the same as a structural formula of the ionic liquid in the mixed liquid (B) before the extraction.
[11] The method for recovering an ionic liquid according to any one of [1] to [10], in which a temperature for extracting the mixed liquid (B) is from 0 to 100°C, from 1 to 90°C, from 3 to 80°C, or from 5 to 70°C.
[12] The method for recovering an ionic liquid according to any one of [1] to [11], in which a pressure for extracting the mixed liquid (B) is from 10 to 2000 hPa, from 100 to 1500 hPa, or from 500 to 1200 hPa (e.g., atmospheric pressure).
[13] The method for recovering an ionic liquid according to any one of [1] to [12], in which the ionic liquid is a salt composed of a cation component and an anion component.
[14] The method for recovering an ionic liquid according to any one of [1] to [13], in which the ionic liquid contains, as a cation component, at least one selected from the group consisting of an imidazolium cation, a pyridinium cation, a pyrrolidinium cation, a piperidinium cation, a quaternary ammonium cation, and a quaternary phosphonium cation.
[15] The method for recovering an ionic liquid according to any one of [1] to [14], in which the ionic liquid contains the imidazolium cation or the quaternary ammonium cation as the cation component.
[16] The method for recovering an ionic liquid according to [14] or [15], in which the imidazolium cation is a cation represented by Formula (1) where R¹ and R³ are, identically or differently, substituted or unsubstituted alkyl groups, alkenyl groups, alkoxyalkyl groups, or substituted or unsubstituted phenyl groups, and R², R⁴, and R⁵ are, identically or differently, hydrogen atoms, substituted or unsubstituted alkyl groups, alkenyl groups, alkoxyalkyl groups, or substituted or unsubstituted phenyl groups.
[17] The method for recovering an ionic liquid according to any one of [14] to [16], in which the pyridinium cation is a cation represented by Formula (2) where R⁶ is an alkyl group, an alkenyl group, an alkoxyalkyl group, or a substituted or unsubstituted phenyl group, and R⁷ to R¹¹ are, identically or differently, hydrogen atoms, alkyl groups, alkenyl groups, alkoxyalkyl groups, or substituted or unsubstituted phenyl groups.
[18] The method for recovering an ionic liquid according to any one of [14] to [17], in which the pyrrolidinium cation is a cation represented by Formula (3) where R¹² and R¹³ are, identically or differently, alkyl groups, alkenyl groups, alkoxyalkyl groups, or substituted or unsubstituted phenyl groups, and R¹⁴ to R²¹ are, identically or differently, hydrogen atoms, alkyl groups, alkenyl groups, alkoxyalkyl groups, or substituted or unsubstituted phenyl groups.
[19] The method for recovering an ionic liquid according to any one of [14] to [18], in which the piperidinium cation is a cation represented by Formula (4) where R²² and R²³ are, identically or differently, alkyl groups, alkenyl groups, alkoxyalkyl groups, or substituted or unsubstituted phenyl groups, and R²⁴ to R³³ are, identically or differently, hydrogen atoms, alkyl groups, alkenyl groups, alkoxyalkyl groups, or substituted or unsubstituted phenyl groups.
[20] The method for recovering an ionic liquid according to any one of [14] to [19], in which the quaternary ammonium cation is an ammonium cation represented by Formula (5) where R³⁴ to R³⁷ are, identically or differently, alkyl groups, alkenyl groups, alkoxyalkyl groups, or substituted or unsubstituted phenyl groups.
[21] The method for recovering an ionic liquid according to any one of [14] to [20], in which the quaternary phosphonium cation is a phosphonium cation represented by Formula (6) where R³⁸ to R⁴¹ are, identically or differently, alkyl groups, alkenyl groups, alkoxyalkyl groups, or substituted or unsubstituted phenyl groups.
[22] The method for recovering an ionic liquid according to any one of [1] to [21], in which the ionic liquid contains, as the anion component, at least one selected from the group consisting of a halogen anion, a pseudohalogen anion, a carboxylate anion, a phosphate-based anion, an amino acid anion, phenolate, pyrimidinolate, a tetrafluoroborate ion (BF⁴⁻), a sulfomethyl ion (CH₃SO₃⁻), methylphosphonate, a sulfate ion, and PF₆⁻.
[23] The method for recovering an ionic liquid according to any one of [1] to [22], in which the ionic liquid contains the carboxylate anion as the anion component.
[24] The method for recovering an ionic liquid according to any one of [1] to [23], in which the organic acid is a carboxylic acid, a C₁₋₁₈ carboxylic acid, a C₁₋₁₀ carboxylic acid, a C₁₋₆ carboxylic acid, a C₂₋₄ carboxylic acid, a C₉₋₁₈ carboxylic acid, and/or a C₁₋₇ carboxylic acid.
[25] The method for recovering an ionic liquid according to any one of [1] to [24], in which the organic acid is at least one type selected from the group consisting of formic acid, acetic acid, propionic acid, and trifluoroacetic acid.
[26] The method for recovering an ionic liquid according to any one of [1] to [25], in which the organic acid is carboxylic acid, and a C₁₋₆ carboxylic acid content relative to the carboxylic acid (total amount) is 70 wt.% or more, 80 wt.% or more, 90 wt.% or more, 95 wt.% or more, or 99 wt.% or more.
[27] The method for recovering an ionic liquid according to any one of [1] to [26], in which a molar ratio of the ionic liquid to the organic acid (ionic liquid:organic acid) contained in the mixed liquid (A) or the mixed liquid (B) is 1:100 to 100:1, 10:90 to 90:10, 20:80 to 80:20, or 30:70 to 70:30.
[28] The method for recovering an ionic liquid according to any one of [1] to [27], in which the amount of the water used or the amount of the water contained in the mixed liquid (A) is 0.01 mol or more, 0.1 mol or more, 1 mol or more, 5 mol or more, 10 mol or more, 15 mol or more, 20 mol or more, 25 mol or more, 1000 mol or less, 500 mol or less, 300 mol or less, 250 mol or less, 200 mol or less, 150 mol or less, 100 mol or less, 75 mol or less, 50 mol or less, 40 mol or less, 0.01 to 1000 mol, 0.1 to 500 mol, 1 to 300 mol, and/or 25 to 40 mol relative to 1 mol of the ionic liquid.
[29] The method for recovering an ionic liquid according to any one of [1] to [28], in which the organic solvent (Or) is at least one type selected from the group consisting of ethers, ketones, esters, aryls, alkanes, and cycloalkanes.
[30] The method for recovering an ionic liquid according to any one of [1] to [29], in which the organic solvent (Or) is at least one type selected from the group consisting of ethers, ketones, and esters.
[31] The method for recovering an ionic liquid according to any one of [1] to [30], in which the organic solvent (Or) is an ether (particularly a dialkyl ether) or an ester.
[32] The method for recovering an ionic liquid according to any one of [1] to [31], in which the ether is at least one selected from the group consisting of a dialkyl ether (diethyl ether, diisopropyl ether, or methyl tert-butyl ether) and dioxane; the ketone is at least one type selected from the group consisting of methyl ethyl ketone, methyl isobutyl ketone, cyclohexanone, and acetophenone; the ester is at least one type selected from the group consisting of methyl acetate, ethyl acetate, butyl acetate, and isopropyl acetate; the aryl is at least one type selected from the group consisting of benzene, toluene, xylene, methoxybenzene, and ethylbenzene; the alkane is at least one type selected from the group consisting of n-hexane and n-heptane; and/or the cycloalkane is cyclohexane.
[33] The method for recovering an ionic liquid according to any one of [8] to [32], in which the amount of the organic solvent (Or) is 1 part by weight or more, 10 parts by weight or more, 20 parts by weight or more, 30 parts by weight or more, 50 parts by weight or more, 10000 parts by weight or less, 6000 parts by weight or less, 5000 parts by weight or less, 4000 parts by weight or less, 2000 parts by weight or less, 1 to 10000 parts by weight, 10 to 6000 parts by weight, 20 to 5000 parts by weight, 30 to 4000 parts by weight, and/or 50 to 2000 parts by weight relative to 100 parts by weight of the total amount of the ionic liquid, the organic acid, and the water contained in the mixed liquid (A).
[34] The method for recovering an ionic liquid according to any one of [8] to [33], in which the mixed liquid (A) further contains a solvent (solvent (T)) in addition to the ionic liquid, the organic acid, the water, and the organic solvent (Or).
[35] The method for recovering an ionic liquid according to [34], in which the solvent (T) is at least one type selected from the group consisting of sulfoxides, sulfones, amides, and lactams.
[36] The method for recovering an ionic liquid according to [34] or [35], in which the solvent (T) is at least one type selected from the group consisting of dimethyl sulfoxide (DMSO), sulfolane, N,N-dimethylformamide (DMF), and N-methyl-2-pyrrolidone (NMP), at least one type selected from the group consisting of N,N-dimethylformamide (DMF) and N-methyl-2-pyrrolidone (NMP), or N-methyl-2-pyrrolidone (NMP).
[37] The method for recovering an ionic liquid according to any one of [34] to [36], in which the content of the solvent (T) contained in the mixed liquid (A) is 0.01 wt.% or more, 0.05 wt.% or more, 0.1 wt.% or more, 0.3 wt.% or more, 0.5 wt.% or more, 30 wt.% or less, 20 wt.% or less, 10 wt.% or less, 5 wt.% or less, and/or 4 wt.% or less.
[38] The method for recovering an ionic liquid according to any one of [34] to [37], in which the content of the solvent (T) contained in the mixed liquid (B) is 1 wt.% or more, 3 wt.% or more, 5 wt.% or more, 10 wt.% or more, 15 wt.% or more, 90 wt.% or less, and/or 80 wt.% or less.
[39] The method for recovering an ionic liquid according to any one of [1] to [38], in which the organic acid is a C₁₋₇ carboxylic acid.
[40] A composition containing a water-soluble ionic liquid, an organic acid, water, and an organic solvent (excluding the organic acid).
[41] The composition according to [40], which is for extraction.
[42] The composition according to [40] or [41], in which the ionic liquid of the composition is at least one type selected from the group consisting of 1-ethyl-3-methylimidazolium acetate (EmimOAc) and 1-ethyl-2,3-dimethylimidazolium acetate (EDmimOAc), or 1-ethyl-3-methylimidazolium acetate (EmimOAc).
[43] The composition according to any one of [40] to [42], in which the organic acid of the composition is acetic acid.
[44] The composition according to any one of [40] to [43], in which the organic solvent of the composition is at least one type selected from the group consisting of ethers, ketones, and esters, or at least one type selected from the group consisting of ethers (particularly dialkyl ethers) and esters.
[45] The composition according to any one of [40] to [44], further containing at least one type selected from the group consisting of dimethyl sulfoxide, N,N-dimethylformamide, and N-methyl-2-pyrrolidone, at least one type elected from the group consisting of N,N-dimethylformamide and N-methyl-2-pyrrolidone, or N-methyl-2-pyrrolidone.

### Industrial Applicability

According to the method for recovering an ionic liquid of the present disclosure, it is possible to easily and efficiently recover the ionic liquid from a mixed liquid containing the ionic liquid and an organic acid.

## Claims

1. A method for recovering an ionic liquid from a mixed liquid containing the ionic liquid and an organic acid, wherein
the ionic liquid is water-soluble, and
the method comprises blending at least one type selected from the group consisting of water and an organic solvent (excluding the organic acid) in the mixed liquid, and separating the organic acid by extraction.

2. The method for recovering an ionic liquid according to claim 1, wherein the ionic liquid contains an imidazolium cation or a quaternary ammonium cation as a cation component.

3. The method for recovering an ionic liquid according to claim 1 or 2, wherein the ionic liquid contains a carboxylate anion as an anion component.

4. The method for recovering an ionic liquid according to any one of claims 1 to 3, wherein the organic acid is carboxylic acid.

5. The method for recovering an ionic liquid according to any one of claims 1 to 4, wherein the organic solvent is at least one type selected from the group consisting of ethers, ketones, esters, aryls, alkanes, and cycloalkanes.

6. The method for recovering an ionic liquid according to any one of claims 1 to 5, wherein the organic solvent is at least one type selected from the group consisting of ethers, ketones, and esters.

7. The method for recovering an ionic liquid according to any one of claims 1 to 6, wherein the organic acid is a C₁₋₇ carboxylic acid.
